# EUROPEAN PATENT APPLICATION

(11) **EP 4 640 195 A1**
(43) Date of publication of application: **29.10.2025**
(21) Application number: 25168945.1
(22) Date of filing: 07.04.2025
(51) Int. Cl.: A61F 11/00, A61F 13/12, A61F 5/01, A61F 13/00

(54) **AESTHETIC DEVICE FOR REDUCING THE DIVERGENCE BETWEEN THE EAR AND THE HEAD**

(30) Priority: 22.04.2024 IT 202400009139
(71) Applicant: Cavalli, Lorenza, 20154 Milano (IT); Gazzina, Alessandro Andrea, 20154 Milano (IT)
(72) Inventor: Cavalli, Lorenza, 20154 Milano (IT)

(57) **Abstract**

The present invention relates to an adhesive aesthetic device (1) designed to be positioned between the ear (2) and the head (3), suitable for reducing the divergence between the two in a stable, comfortable, discreet, and lasting manner for at least 48 hours, without causing trauma or leaving residues upon removal, while maintaining the space (5) between the ear and the head free, allowing for the stable placement of commonly used objects.

The invention, due to its particular structure, features a curved body (6) and two fixing wings (10, 11), the body having a fixed and non-modifiable rigidity that keeps the amplitude of the curvature (9) and the distance (5) between the two fastening wings (10, 11) constant and unchanged, even under pushing or pulling forces applied before or during application. This is achieved without the need for connecting elements occupying the space between the ear and the head, and prevents the adhesive layer from folding onto itself during the delicate application phase, thereby preserving its adhesive capacity. The thinned edges of the body at the fastening wings provide them with slight flexibility, which enhances wearing comfort and contributes to the adherence and stability of the device. The device has an adhesive surface (7) on the side intended for attachment to the skin.

## Description

### BACKGROUND OF THE INVENTION

Prominent ears, also known as protruding ears, although not necessarily impairing functional or auditory performance, can nevertheless be the cause of significant problems and discomfort, including:
- *Reduced self-esteem,* especially during childhood and adolescence when individuals are more sensitive to the judgment of others;
- *Bullying,* as such a characteristic may become a target for prejudice from peers or other individuals, resulting in emotional and psychological issues, sometimes of considerable severity;
- *Difficulty using earphones,* which may not fit properly or may be uncomfortable to wear due to the shape and angle of the ears;
- *Greater risk of injury,* as prominent ears may be more exposed to trauma during certain physical activities or situations where there is a risk of impacts to the head.

Beyond the first years of life-when cartilage is still malleable-a permanent solution to this issue is only achievable through surgical intervention.

Besides the economic burden associated with surgery, this condition is sometimes perceived as a manageable complex that can be addressed with the economical, non-invasive, and reversible solution proposed by the present invention.

The innovative contribution of the device, suitable for both adults and children, lies in its unique structure, which maintains a defined distance between the ear and the head, correcting the divergence in an effective, discreet, long-lasting, and significantly comfortable manner, without occupying the space needed for the stable placement of commonly used objects.

This invention offers a solution applicable across multiple fields:
- *Aesthetic field,* where the excessive distance or closeness between the head and the auricle may cause psychological discomfort;
- *Entertainment and stage arts,* where it may serve as a useful tool for camouflage and makeup, especially when it is necessary to alter the angle between the ears and the head;
- *Sports field,* where the insertion of items between the ears and the head may be helpful or necessary, such as glasses, helmets, headbands, thermal or sweat-absorbing bands;
- *Professional settings,* where the use of face masks, uniforms including accessories worn behind the ears, or specific technological devices such as cameras, transmitters, or sensors may be more stable, functional, or comfortable when positioned in that area.

### STATE OF THE ART

The state of the art addressing the need to modify the divergence between the ears and the head in both adults and children includes the following types of solutions:
- *Irreversible solutions, such as surgical intervention;*
- *Solutions that fully or partially occupy the space between the ear and the head, thus impeding the placement of commonly used objects;*
- *Solutions in which the device tends to fold onto itself during application, compromising the adhesive layer;*
- *Solutions that do not provide a realistic or natural-looking correction;*
- *Solutions that shift position during use due to poor stability or inconsistent adherence;*
- *Solutions that act on the shape of the auricle without reducing the distance between the ear and the head;*
- *Solutions requiring pushing or pulling forces to position the device;*
- *Solutions in which the adhesive power is not supported by principles of anatomical adherence or conformability;*
as exemplified by the following prior art documents:
EP2100574A1; US2002/062110A1; US2014/224568A1; WO2012100121A2; WO9409731A1;
US5076262A; ES1231511U; WO2016153393A1; FR2743718A1; US2010059078A1; US4187838A; ITMI20092200A1.

The device of the present invention is an innovative system that overcomes the drawbacks of prior art solutions aimed at modifying the distance between the head and the ear, both with and without surgical intervention.

The key features that address the above disadvantages are as follows:
- *Rigidity and shape retention:* The device comprises a rigid body with a fixed, constant, and non-modifiable curvature, which preserves its shape and the distance between the two fixing wings even when subjected to pushing or pulling forces applied before or during application. In addition to maintaining free the space between the ear and the head for the stable insertion of commonly used objects, this ensures more stable and consistent adherence compared to devices prone to deformation or positional changes during use. Furthermore, it prevents the adhesive from folding during the critical application phase, where other devices often bend and become unusable due to compromised adhesion.
- *Tapered edges:* In addition to having rounded margins that recede in correspondence with the curvature-allowing the device to adhere more effectively and comfortably to the junction area between the ear and the head-and extend outward toward the ends to increase the fixing area, the distinctive feature of these margins (unaddressed by prior art) is their tapering at the wings. This tapering provides slight flexibility that enhances comfort during use and contributes to better adherence and stability of the device.
- *Converging fixing wings:* The particular converging orientation of the wings, which maintains a wider spacing near the curvature and gradually narrows toward the ends, results in a more concealed and discreet correction, and thereby more effective. It also leaves more space available for the stable placement of commonly used objects such as eyeglasses, hearing aids, headphones, headbands, and various ornamental items. This feature further contributes to a more even distribution of pressure across the two ends of the device, thereby increasing the overall efficiency of the invention.
- *Specific materials and coatings:* The ability to use certain materials and coatings-detailed below-provides additional advantages in terms of comfort, durability, hygiene, and adherence, compared to materials conventionally used in other devices.
- *Additional functional elements:* Optional, non-limiting features such as the self-molding adhesive surface, which conforms perfectly to the protrusions and recesses resulting from the anatomical peculiarities of different users, increase both the adhesive power and comfort during prolonged wear. The presence of a layer for the gradual release of fragrances or essential oils with calming, relaxing, or revitalizing properties-whether applied by the user prior to use or pre-integrated into the device (possibly coinciding with the self-molding layer or separate from it)-enhances the sense of well-being during use.

This invention thus overcomes the limitations of the prior art by offering an aesthetic correction that is economical, effective, reversible, adaptable to different anatomical structures, significantly comfortable, virtually invisible, and capable of holding firmly and durably for at least 48 hours, all while keeping the space between the ears and the head free for stable object insertion. Furthermore, some adhesive devices in the prior art require the exact placement position to be determined before application, since even a single reapplication would compromise the adhesive power.

Advantageously, thanks to the fixed rigidity of the device's body and its shape with receding, extending, and tapered margins, the invention can adapt to most areas of application and, once applied, remains firmly in place even when objects are inserted in the space between the ear and the head.

### TECHNICAL DESCRIPTION

The subject matter of the present invention is characterized by a shaped body (6) with a fixed, non-modifiable curvature (9) and tapered ends.

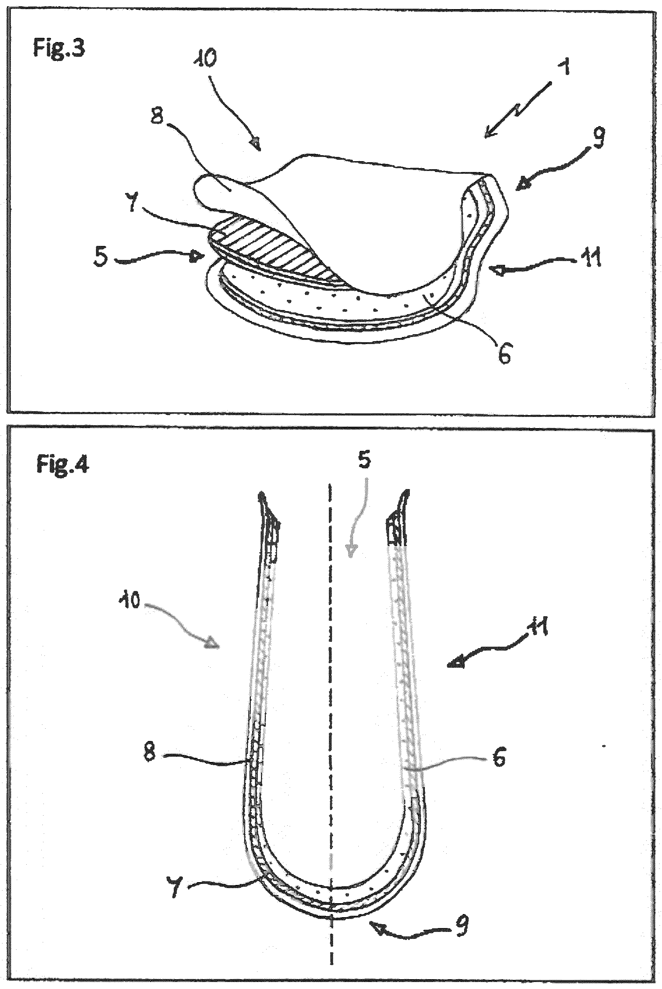

The curvature, located at or near the symmetrical axis of the body (see Fig. 4), provides a convexity (9) designed to adhere to the anatomical junction area (4) between the ear (2) and the head (3). It features a thickness with a given fixed rigidity which, extending along the body (6) of the device, gradually decreases toward the ends, while maintaining both the curvature width and the distance between the two fastening wings (10 and 11) constant and unchanged. Despite this, the wings maintain slight flexibility with respect to the convexity (9), which enhances user comfort and the adhesion of the device.

Said fixed rigidity of the body (6), which maintains the distance between the fastening wings (10 and 11) even when push or pull forces are applied before or during use, makes any connecting elements between the wings unnecessary. The absence of such connecting elements in the divergence space (5) between the ear and the head not only allows the insertion of common-use objects, but also renders the correction nearly invisible and thus very discreet.

The two fastening wings, one (10) to the ear (2) and the other (11) to the head (3), have a converging trajectory toward the ends. This feature keeps the space (5) wider near the curvature (9), facilitating the insertion of everyday objects, and narrower toward the ends, so that by tilting the outer margins of the ear toward the user's head, the correction becomes more concealed and therefore more effective. The fixed-curvature shape of the device maintains a distance (5) between the wings (10 and 11) that may vary from a few millimeters to 1 cm, depending on the size version, thereby allowing aesthetic correction to be adapted to the needs of both adults and children.

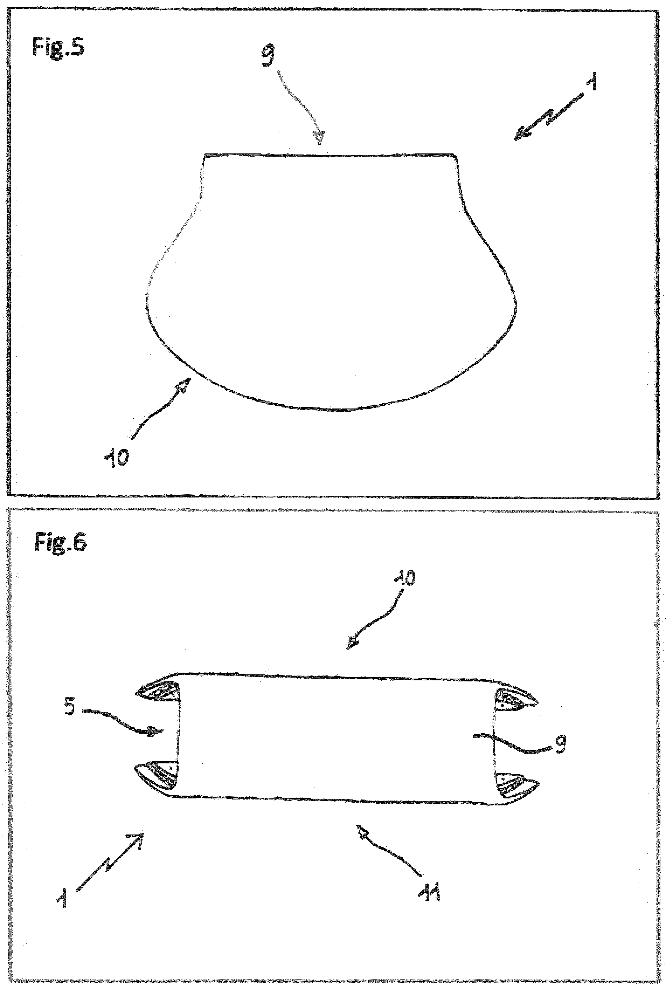

In a suggested, non-limiting embodiment, the contour of the invention (see Figures 3, 5 and 6) features a profile with rounded edges that recede at the curvature (9) and protrude at the fastening wings (10 and 11), thereby providing greater adaptability to the junction area (4) between the ear and the head, and a wider contact surface at the attachment areas (10 and 11) of the ear (2) and the head (3).

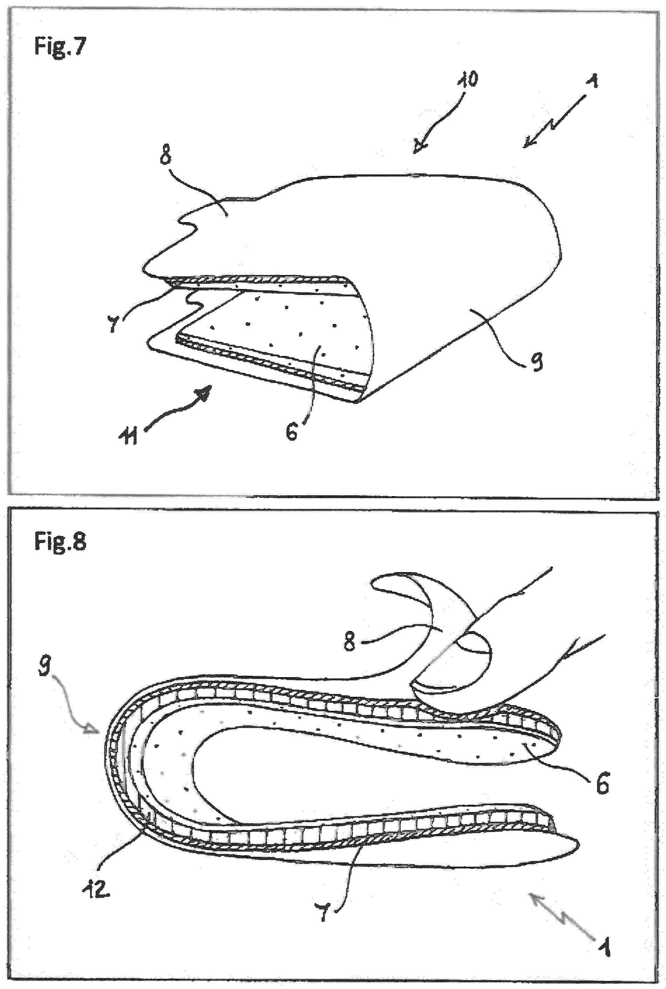

In a preferred but non-limiting embodiment, the surface of the body of the device facing the ear and head may include a self-molding layer (12 in Fig. 8) of a defined malleable and conformable thickness, which is either adhesive or covered with glue. This feature, in addition to making the device more comfortable to use, allows for more effective adhesion, ensuring greater stability and minimizing the risk of undesired shifting or detachment during use.

The portion of the device intended to come into contact with the skin is coated with a hypoallergenic adhesive surface (7), designed to ensure stable and lasting adhesion for at least 48 hours, without causing trauma or leaving residues on the skin upon removal. This adhesive surface may cover the entire area of the device designated for skin contact or only the surfaces corresponding to the fastening wings (10 and 11).

Furthermore, the adhesive surface may cover the self-molding layer (12, Fig. 8) or coincide with it, featuring its own malleable thickness and ability to conform to different ear morphologies, without reducing the traction force exerted by the adhesive.

The adhesive area (7) is covered by a shaped, protective, and removable film (8), which may extend beyond certain portions of the device body (6) to allow for easier handling by the user without compromising the adhesive's effectiveness (Fig. 7).

In a further possible, non-limiting embodiment, the body (6) of the device may include a layer of material capable of gradually releasing fragrances or essential oils. This layer may coincide with or be independent from the adhesive self-molding surface (12), adding further comfort to the device.

Said fragrances or essential oils may already be incorporated into this layer or applied by the user before use.

The addition of a material layer to the device body that gradually releases calming, relaxing, or invigorating fragrances or essential oils may enhance the user's comfort and well-being during use, addressing a variety of needs, including:
- *Aromatherapy:* the release of essential oils may provide benefits for both physical and mental regeneration.
- *Odor masking:* use during sports activities or physical exertion may help mask potential body odors with pleasant fragrances.
- *Mood enhancement:* such scents may positively influence the user's emotional state.

The ability to apply fragrances or essential oils selected directly by the user before use also allows the device to be adapted to the individual olfactory preferences and intensity sensitivities of different users. Additionally, the option of applying fragrance to the area behind the ears is a fairly common practice, often due to personal preferences, cultural habits, and individual beliefs, including:
- *Pulse point:* the area behind the ears is considered a pulse point, where arteries are closer to the surface of the skin. Some individuals believe that applying fragrance in this area helps its diffusion, as the body heat enhances evaporation.
- *Fragrance retention:* it is often believed that applying essential oils or perfumes behind the ears helps retain the scent longer than on other areas like the wrists or neck, as the skin in that area is generally less exposed to environmental factors and may therefore preserve the fragrance longer.
- *Cultural or personal habits:* in some cultures or communities, applying perfume behind the ears is a common or traditional practice; in other cases, it is simply a personal preference based on individual experience or fragrance application routines.
- *Gesture breadth:* applying perfume behind the ears may be part of a broader application ritual. Some people apply fragrance to multiple body points for a more diffused and lasting scent, with the area behind the ears being one of them.

### TECHNICAL DETAILS OF THE MATERIAL

The material of the body (6) of the device (1) features a defined thickness with a fixed, stable, constant, and non-modifiable rigidity, with tapered edges at the ends of the fastening wings (10 and 11) (see Fig. 4).

This body (6) may be transparent or colored in various shades, including tones matching different skin colors. It is made from a hypoallergenic, breathable, and waterproof material, selected from among plastics, thermoplastics, antibacterial or thermoregulating materials, polymers, rubbers, resins, or silicone.

The advantages associated with the use of the proposed materials include:
- *Plastic materials:*
   - Flexibility: can be designed to offer a certain degree of flexibility, useful for creating ergonomic and comfortable devices.
   - Resistance: many types of plastic offer good resistance to wear and impacts, ensuring the durability of the device.
   - Lightweight: often lightweight materials, making the device highly comfortable to wear.
- *Thermoplastic materials:*
   - Moldability: become malleable when heated, thus facilitating the production of specific shapes and contours.
   - Ease of processing: can be easily melted and formed, making the manufacturing process more efficient.
- *Antibacterial materials:*
   - Hygiene: the addition of antibacterial agents can help prevent the growth of bacteria on the device, reducing the risk of infections or contamination.
- *Thermoregulating materials:*
   - Comfort: can help maintain a comfortable temperature on the surface of the device, reducing the risk of overheating or excessive cooling.
   - Adaptability: especially suitable for wearable devices, as they help maintain a constant temperature near the body.
- *Polymeric materials:*
   - Versatility: polymers can be engineered with a wide range of properties, such as transparency, flexibility, or rigidity, to meet specific design requirements.
- *Rubbers:*
   - Shock absorption: can provide protection against impacts, helping to safeguard both the device and the user.
- *Resins:*
   - Durability: highly durable and wear-resistant, contributing to the device's longevity.
- *Silicone:*
   - Hygiene: commonly used for its non-porous and bacteria-resistant nature, contributing to the cleanliness and hygiene of the device while reducing the risk of skin irritation.
   - Comfort: provides a certain softness and flexibility, making the device particularly comfortable to wear.

The introduction of the wide range of listed materials allows for the creation of a more comfortable, adaptable, and durable product, thereby improving the overall user experience of the invention.

As specifically described, some of the listed materials, such as silicone or resins, can provide increased comfort, durability, and longevity of the device, while others can offer slight flexibility. This may allow for better adaptation to the shape of the user's ear and head, without compromising the fixed rigidity of the device body in maintaining the curvature width and the distance between the wings. The use of antibacterial material for both the body of the device and the adhesives helps reduce the risk of skin infections and improves hygiene during prolonged use.

Among the most widely used materials with antibacterial properties are:
- *Copper and copper alloys:* copper has demonstrated antibacterial and antimicrobial properties, with a high capacity to kill bacteria and inhibit microbial growth.
- *Zinc:* zinc oxide is known for its antibacterial properties and is often used in creams and ointments for skin care. Zinc coatings are also applied in some fabrics for their antimicrobial properties.
- *Silver:* silver is another metal with strong antibacterial properties. It is used in various products, including wound dressings, plasters, and medical devices.
- *Bamboo:* bamboo fabric has been shown to possess natural antibacterial properties. It is used, among other products, in the manufacture of textiles and underwear suitable for contact with sensitive and delicate skin.
- *Antibacterial plastic:* some polymers have been modified to make the material resistant to bacteria and pathogens. These materials may be used for surface coatings, food packaging, and medical devices.
- *Fabrics treated with antibacterial substances:* certain textiles are treated with antibacterial agents during the manufacturing process to prevent unwanted odors and reduce bacterial growth.

The choice to use thermoregulating materials in the body of the device to maintain a constant and comfortable temperature on the surface in contact with the skin enhances comfort during prolonged use. Thermoregulating materials are specifically designed to adjust temperature in response to environmental or body temperature variations.

These materials may have the ability to absorb, release, or reflect heat to maintain a stable and comfortable temperature. They are used in various applications, including technical clothing, thermal insulation, home appliances, and medical devices.

In a non-limiting embodiment, the surface of the body (6) of the device intended for contact with the skin features a self-molding surface (12) with a malleable and conformable thickness that may be adhesive or coated with glue (7). This self-molding surface is made from a flexible, elastic, soft, waterproof, and hypoallergenic material, selected from among: expanded polyethylene, plastic and thermoplastic materials, synthetic materials, antibacterial or thermoregulating substances, polyurethane foams, memory foam, rubbers, polyvinyl chloride, silicone, non-woven rayon fibers, non-woven fabric, or hydrocolloid materials.

The possibility of manufacturing this surface from a variety of materials allows the device to optimally adapt to the shape and sensitivity of the user's skin.

Overall, the combination of the listed materials and their specific characteristics may help ensure a more comfortable, safe, and effective user experience with the device.

The main advantages of these materials are:
- *Expanded polyethylene:* lightweight, flexible, and water-resistant. It can also provide a certain level of cushioning and impact protection.
- *Plastic and thermoplastic materials:* may offer a combination of strength, flexibility, and workability, allowing for more precise and customized device design.
- *Polyurethane foams and memory foam:* these materials may offer high levels of comfort, evenly distribute pressure on the skin, and help prevent skin irritation.
- *Rubbers and polyvinyl chloride (PVC):* may provide flexibility and wear resistance, contributing to the durability and functionality of the device.
- *Silicone:* its non-porous nature and resistance to bacterial agents make it ideal for sensitive skin and for ensuring secure and comfortable adhesion.
- *Non-woven rayon fibers and non-woven fabric:* may provide a soft and breathable surface, reducing the risk of skin irritation or allergic reactions.
- *Hydrocolloid materials:* may offer moisture-absorbing and skin-protective properties, reducing the risk of irritation due to humidity or friction.

The adhesive surface (7), which may also cover or coincide with the self-molding layer (12), can be applied to the entire surface of the device intended for skin contact or, alternatively, only to the fastening wings (10 and 11), one affixed to the ear (2) and the other to the head (3). This configuration leaves the convexity (9), which is intended to adhere to the junction area (4) between the ear and the head, free of adhesive.

The glue or adhesive compound applied at least to the surfaces of wings (10 and 11) designed for skin adhesion is selected from among hypoallergenic, biocompatible, epidermal, antibacterial, thermoplastic, thermoregulating, acrylate, and silicone adhesives. These may be non-water-soluble, water-repellent, non-staining to the skin, and possess high adhesion strength, with a stable hold of at least 48 hours, without causing trauma or leaving residues on the skin upon removal.

The wide selection of proposed adhesives allows for the production of the aesthetic device tailored to specific and preferred user needs, focusing on certain aspects related to the overall efficacy and usability of the invention.

The various advantages of this range of options include:
- *Skin safety:* the use of hypoallergenic and biocompatible adhesives reduces the risk of allergic reactions or skin irritation, ensuring that the device can be used safely even on sensitive skin. Antibacterial adhesives reduce the risk of skin infections and improve hygiene during prolonged use of the device.
- *Comfort:* epidermal adhesives are designed to adhere gently to the skin without causing discomfort or irritation, ensuring a comfortable experience for the user.
   Thermoregulating skin adhesives are designed to regulate body temperature through skin contact and are made of materials with specific thermal properties to absorb or release heat depending on the user's needs, offering particularly comfortable experiences.
- *Resistance and durability:* adhesives such as thermoplastic, acrylate, or silicone types can offer greater strength and long-term performance, ensuring that the device remains securely in place for extended periods without requiring reapplication.
- *Adaptability:* the use of non-water-soluble and water-repellent adhesives ensures that the device maintains its adhesion even in the presence of moisture or sweat, offering greater reliability in various environmental conditions.

The adhesive surfaces (7) are covered by at least one shaped and removable protective sheet (8), made from a material selected from plastic materials, metallic materials, siliconized polyester, siliconized paper, or polyethylene-coated paper.

The choice of material will depend on specific requirements related to protection, flexibility, and transparency.

In one possible embodiment (Fig. 7), the protective sheets (8) include, in certain areas, an extended surface that exceeds the adhesive area (7), in order to facilitate the application of the device without compromising its adhesive strength through direct contact.

The specific advantages associated with the use of the different proposed materials for the protective sheets (8) include:
- *Plastic materials:*
   - Flexibility: allows the protective sheet to easily adapt to the shape of the patch.
   - Waterproofing: protects the adhesive surface from moisture, water, and other liquids.
   - Tear resistance: resists tearing, helping to preserve the integrity of the adhesive surface.
- *Metallic materials:*
   - Puncture resistance: provides extra protection against accidental punctures or perforations.
   - Heat barrier: can shield the patch from external heat sources.
   - Durability: may retain structural integrity for a longer period compared to some plastic materials.
- *Siliconized polyester:*
   - Smooth surface: reduces friction and facilitates removal of the protective sheet.
   - Transparency: allows the adhesive surface of the patch to be easily visible.
   - Moisture resistance: protects the adhesive from humidity and sweat.
- *Siliconized paper:*
   - Biodegradability: more eco-friendly than many other materials and can be disposed of more sustainably.
   - Conformability: adapts well to the adhesive surface of the patch.
   - Cost-effectiveness: may be a more economical option compared to other materials.
- *Polyethylene-coated paper:*
   - Tensile and tear resistance: provides good resistance to tension and tearing.
   - Waterproofing: effectively protects the adhesive surface from moisture and liquids.
   - Flexibility: bends and conforms to the shape of the patch without compromising its integrity.

In a possible, non-limiting embodiment, the body of the device may house or be covered by a layer of material capable of gradually releasing fragrances or essential oils. These may have calming, relaxing, or invigorating properties and can either be already incorporated within the layer or applied by the user before use.

The layer for gradual fragrance release may be colored or transparent, and is made of fabric or non-woven material, comfortable on the skin and suitable for retaining fragrance.

The scented substance may consist of essential oils, synthetic perfumes, or other aromatic compounds, which may be encapsulated or impregnated into the material.

If the device is designed to receive the fragrance directly from the user before use, the layer intended to hold the aromatic substance may be made from a porous or absorbent material that retains and gradually releases the fragrance. This material may consist of non-woven fabric, absorbent paper, or a blend of porous polymers.

Whether the layer already contains the fragrance or is designed to receive it before use, it may coincide with or be separate from the self-molding surface (12) and, in either case, is covered by the protective sheet (8) to be removed before application.

The subject of the invention is easy and cost-effective to industrialize.

### APPLICATION

Once the preferred application area between the ear (2) and the head (3) has been identified-ensuring the area is clean, dry, and free of sebum or hair-the protective sheets (8) covering the adhesive surfaces (7) are removed, and the device (1) is applied to the selected area by gently pressing it, so that the device adheres fully to the skin.

The corrector will remain firmly in place for at least 48 hours, even under repeated pulling or pressure on the ear, or during the insertion of everyday objects into the space (5) between the ear and the head. Once use is complete, the device can be easily removed by lifting any portion of the surface in contact with the skin, without causing trauma or leaving residue on the skin.

In case of slight adhesive residue, it can be easily removed with a small amount of water.

### BRIEF DESCRIPTION OF THE FIGURES

The invention is described below with reference to the accompanying figures, which illustrate some non-limiting embodiments thereof, in which:

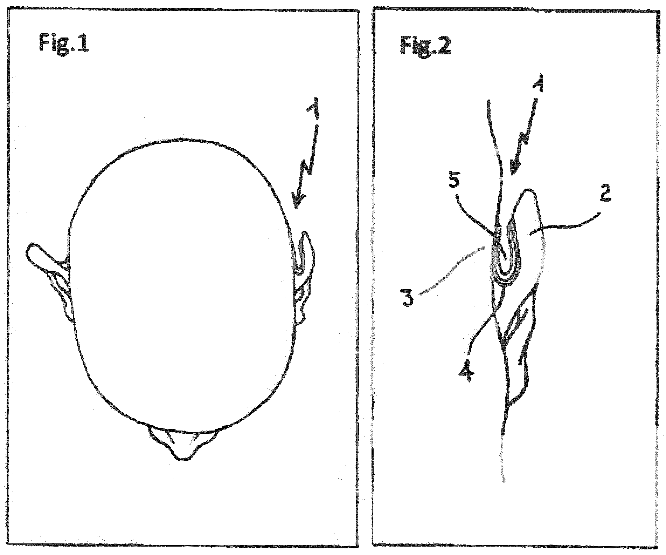

- *Figures 1 and 2* are top views of the device applied in accordance with the present invention, where Figure 1 shows the difference in distance between the ear and the head, with and without the application of the device, and Figure 2 represents a detail of Figure 1 with the device applied;
- *Figure 3* is an axonometric view of the device;
- *Figure* 4 is a sectional view of the device;
- *Figure 5* is a top view of the device;
- *Figure 6* is a rear view of the device;
- *Figure* 7 is an axonometric view of the device in a preferred, non-limiting embodiment, showing the excess areas of the protective sheet relative to the body of the device;
- *Figure 8* is a side view of the device in a preferred, non-limiting embodiment, showing the conformability of the self-molding surface.

## Claims

1. Aesthetic device (1) for reducing the divergence between the ear (2) and the head (3), **composed of** a curved body (6), whose convexity is predisposed to adhere to the junction area between the ear and the head (4), and whose two areas at the opposite ends are predisposed to adhere, one (10) to the posterior portion of the ear (2) and the other (11) to the corresponding portion of the user's head (3), and by an adhesive surface (7) on the side of the device intended for attachment to the skin, **characterized by the fact that** the curved body (6) possesses a rigidity fixed, invariable and such as to maintain a given amplitude of the curvature (9) and a given distance (5) between the two fixing wings (10 and 11), and that said body (6) furthermore presents thinned edges in correspondence to the fixing wings (10 and 11) to the user's head and ear.

2. Aesthetic device according to claim 1 **characterized by the fact that** the wings (10 and 11) have a converging direction through which the distance between them (5) is greater at the curvature (9) and decreases towards the ends.

3. Aesthetic device according to one or more preceding claims, **characterized by the fact that** the body (6) of the device has furthermore rounded edges that recede at the curvature (9) and protrude, tapering off, at the fixing wings (10 and 11).

4. Aesthetic device according to the preceding claims **characterized by the fact that** the body (6) of the device is made of a material chosen from plastics, thermoplastics, antibacterial, thermoregulatory materials, polymers, rubbers, resins, or silicone.

5. Aesthetic device according to one or more preceding claims **characterized by the fact that** at least the faces of the wings (10 and 11) of the device intended for attachment to the skin, one to the ear (2) and the other to the head (3) of the user, are covered with adhesive or an adhesive mixture (7).

6. Aesthetic device according to one or more preceding claims **characterized by the fact that** the adhesive or adhesive mixture (7) covering the surfaces intended for adherence to the skin is chosen from hypoallergenic, biocompatible, epidermal, antibacterial, thermoplastic, thermoregulatory, acrylic, silicone, non-water-soluble, and water-repellent adhesives.

7. Aesthetic device according to one or more preceding claims **characterized by the fact that** the adhesive surfaces (7) are covered by at least one shaped and removable protective sheet (8) made of a material chosen from plastics, metals, silicone-coated polyester, silicone-coated or polyethylene-coated paper.

8. Aesthetic device according to one or more preceding claims **characterized by the fact that,** in a non-limiting embodiment, the face of the body (6) of the device intended for contact with the skin is furthermore covered by a self-modeling surface (12) of a given malleable and conformable thickness (7).

9. Aesthetic device according to claim 8 **characterized by the fact that** the self-modeling surface (12) covering the face of the body (6) of the device intended for contact with the skin is made of a material chosen among expanded polyethylene, plastic and thermoplastic materials, synthetic materials, antibacterial, thermoregulatory, polyurethane foams, memory foam, rubbers, polyvinyl chloride, silicone, non-woven rayon fibers, non-woven fabric, hydrocolloidal materials.

10. Aesthetic device according to one or more preceding claims **characterized by the fact that** the body of the device further comprises a layer of material capable of gradual release of perfumes or essential oils, which can either be already present within said layer or applied by the user before use.
